# EUROPEAN PATENT SPECIFICATION

(11) **EP 4 347 004 B1**
(45) Date of publication and mention of the grant of the patent: **07.05.2025**
(21) Application number: 22727187.1
(22) Date of filing: 11.05.2022
(51) Int. Cl.: A61N 1/372, A61B 5/0538, A61B 5/11, A61B 5/00

(54) **COMMUNICATION SYSTEM AND METHOD FOR AN IMPLANTABLE MEDICAL DEVICE**
KOMMUNIKATIONSSYSTEM UND -VERFAHREN FÜR EINE IMPLANTIERBARE MEDIZINISCHE VORRICHTUNG
SYSTÈME ET PROCÉDÉ DE COMMUNICATION POUR UN DISPOSITIF MÉDICAL IMPLANTABLE

(30) Priority: 01.06.2021 US 202163195405 P; 02.07.2021 EP 21183354
(43) Date of publication of application: 10.04.2024
(73) Proprietor: BIOTRONIK SE & Co. KG, 12359 Berlin (DE)
(72) Inventor: TAFF, Brian M., Portland, Oregon 97217 (US); SWENSON, Kurt, Dayton, Oregon 97114 (US); BERGSTROM, Dean, West Linn, Oregon 97068 (US); MERLIN, Julian, 10779 Berlin (DE); MCMILLAN, Brad, Lake Oswego, Oregon 97035 (US)
(74) Representative: Biotronik Corporate Services SE
(86) International application number: PCT/EP2022/062727
(87) International publication number: WO 2022/253531

(56) References cited:
- EP-A1- 1 174 163
- EP-A1- 1 322 217
- EP-B1- 1 322 217
- US-A1- 2007 167 996

## Description

The invention is directed to a communication system for a wireless message transfer between an implantable medical device (IMD) and an external device wherein the IMD is configured to monitor the health status of a patient and/or configured to deliver a therapy signal to the patient. The external device is located at least partially extracorporeally. The invention is further directed to a respective method for wireless message transfer, respective computer program product and a respective computer readable data carrier. The computer program product may be a software routine, e.g. related to hardware support means within the IMD and/or the external device.

Active and passive implantable medical devices (IMDs), for example, a pacemaker (with leads), a BioMonitor, an Implantable Leadless Pacer (ILP), an Implantable Leadless Pressure Sensor (ILPS), an Implantable Cardiac Defibrillator (ICD) or a Subcutaneous Implantable Cardiac Defibrillator (i.e., S-ICD), contain sensors that collect physiological signals to monitor the health status of the patient and transmit them as data to an physician or patient device, or to a remote server using the external device. The data collected from these various or any such sensors can include, but are not limited to, ECG, impedance, activity, posture, heart sounds, pressure, respiration, and other data. Active IMDs, (e.g., pacemaker, ILP, ICD, or S-ICD) may provide therapy output to the patient, for example electrical stimulation within a heart chamber (e.g., atrium or ventricle).

Usually, such IMDs are comprised of a processor for data processing and a transceiver module configured to bi-directionally exchange messages with the external device if, for example, implanted within the body of the patient. The external device, possibly using its own transceiver, is configured for bi-directionally exchange messages with the transceiver module of the IMD. The external device may be a separate device connected to a computer (in some cases called programmer) or may be a module integrated within a remote device such as a computer. The external device produces and sends messages to the transceiver of the IMD in form of requests, for example for receiving data concerning the health status of the patient or IMD status from the IMD or for programming (in order to configure the IMD to apply appropriate therapies to the patient).

In state-of-the-art IMDs, such as Cardiac Rhythm Management (CRM) implants with leads, external device (e.g. connected to a Programmer) communication with the IMD usually has proceeded without a need for the system to have knowledge of or coordinate with more than a single IMD at a given time. This long-standing convenience unfortunately proves incompatible with the needs for, especially end of service, use cases in leadless IMDs. In leadless contexts, because a common set of leads cannot be swapped between two separate IMDs to deliver "fresh" therapy as a replacement for an "old" IMD, administered at the exact same location within the patient's organ (e.g. the patient's heart) by means of a rapid header connection swap, a communication scheme is needed to support the phase out and phase in of therapy support from "old" and "new" IMDs. Further, if the system is to support, even serial, communication with more than one leadless IMD, it must be true that any single such IMD does not interfere with the external device in ways that would prevent data exchange with any in-range IMD or any other in-range external device.

In cases where legacy active IMDs are resident within the communication range of the external device, receipt of a "wake up" signal (often via high-energy pulses or HEPs) by such legacy active IMDs can, depending on the protocols involved, force transitions to states where the legacy active IMDs immediately begin transmitting response output. Such interactions can create an incessant "spewing" of data to the external device that denies support for other devices to be recognized, effectively sidelining any capacity for communicating with a plurality of IMDs. As prior CRM implant systems never considered conditions where another in-body implant might need to communicate with the external device, there was little to no incentive for any single device not to fully occupy the allowable extents of the communication link. This type of interaction is especially problematic in cases where the carrier rates are designed to be low enough to ease signal transmission though patient anatomy, e.g. 32 kHz, as slower data rates encourage longer transmission and receipt interactions creating a larger target for problematic temporal overlap of any in-range, competing transmissions emergent from other implants.

It should be noted too that in some IMD communication support schemes common to the CRM market there has been a history of using higher-frequency communication carriers (e.g., 200 kHz) to enable more rapid data exchange. While such strategies might, in adapted forms, potentially enable more IMDs to non-competitively interact with an external device (subject to proper system design), the attenuation of higher-frequency communication signaling is increasingly problematic subject to greater physical separations between the implant and the external device. In leadless IMDs (which are located inherently deeper within patient anatomy), adoption of higher-frequency communication strategies unfortunately drives a need for the implants to draw power that they are less well equipped to support - directly challenging IMD longevity for this newly emergent class of products.

In document US 2007/167996 A1 an implantable medical device (IMD) is described including a telemetry module to communicate with an external device according to a given protocol. To establish a communication session, the IMD will extend active periods of reception on a given channel when some confirmed data is received from the external device. In addition, once a session has been opened, the programmer transmits a short data set (or preamble) for each cycle which the IMD is set to receive. This data set indicates whether additional data will or will not be sent. If no additional data is to be sent during that cycle, then the IMD powers down the receiver for that cycle.

In document EP 1322217 A1 an apparatus and a method for a telemetry system are described that automatically selects a symmetric modulation protocol configuration for telemetry communication between medical devices and programmers used in providing patient treatment. The standardized telemetry protocol will use the symmetric modulation protocol configuration to establish a downlink or uplink connection. The standardized telemetry protocol will automatically select the appropriate modulation protocol configuration depending on the modulation format and data rate capability best suited for the components needing to communicate via telemetry.

In document EP 1174163 A1 a telemetry system and a method of monitoring cardiac activity or providing therapy to the heart for an implantable cardiac device are described. The system includes a receiver and a transmitter in an external programmer and a receiver section and transmitter section in the cardiac device. The receiver section and the transmitter section of the cardiac device generally remain in a de-energized state. The receiver section is energized at spaced apart time intervals to detect an initiation signal transmitted by the external programmer. If the initiation signal is detected, the receiver section and the transmitter section of the cardiac device are fully activated. The external programmer includes a signal strength indicating means for providing an indication of received signal strength which the user can use to position the receiver and transmitter in the external programmer for optimum received signal strength. An antenna is disposed in the header of the implanted device to facilitate communication at high speed.

Accordingly, it is desirable to provide a communication system and method that enables a low-overhead means for supporting communication with a plurality of IMDs and facilitating targeted IMD-specific interactions as part of both IMD assembly and clinical use.

The above problem is solved by a communication system for a wireless message transfer between an IMD and an external device with the features of claim 1, by a respective communication method with the features of claim 10, by a computer program product with the features of claim 14, and by a computer readable data carrier with the features of claim 15. The computer program product may be a software routine and/or related hardware support means with the IMD and external device.

In particular, the problem is solved by a communication system for a wireless message transfer between an IMD and an external device, comprising an IMD and an external device, wherein the IMD is configured to monitor the health status of a patient and/or configured to deliver a therapy signal to the patient. The IMD comprises a processor, a memory module and a transceiver module configured to bi-directionally exchange the messages with the external device. The external device is configured to send a predefined wake-up signal to the transceiver module of the IMD combined with an ID request message following the wake-up signal within a predefined first time interval, wherein the ID request message contains one of a predefined set of predefined different request specifications. The processor of the IMD is configured to produce an ID response message to the previously received ID request message to send this ID response message from the transceiver module to the external device within a time slot after the receipt of this ID request message. The ID response message comprises an ID information read from a memory cell section at a predefined memory address of the memory module, wherein the section of the memory cell is determined by the processor based on the request specification sent by the previously received ID request message and the time slot is determined by the processor based on the ID information read from the determined section of the memory cell. The request specifications can also be called ID request commands. Further, the memory cell can also be called memory (without cell).

Alternatively or additionally, the processor of the IMD is configured to produce an ID response message to the previously received ID request message and to send this ID response message from the transceiver module to the external device within a time slot after the receipt of this ID request message, wherein the ID response message comprises an ID information read from a predefined memory address of the memory module, wherein the section of the memory is determined by the processor based on the request specification sent by the previously received ID request message and the time slot is determined by the processor based on the ID information read from the determined section of the memory.

The IMD is an implantable medical device as defined above configured to monitor the health status of a patient and/or configured to deliver a therapy signal to the patient, for example an ILP, an ILPS or an ICD.

The IMD comprises a processor for data processing and a transceiver module (e.g. an antenna or a coil connected to an appropriate communication management capability) for receiving messages (i.e. communication signals) from and transmitting messages to the external device. The request messages are instruction or command requests sent by the external device to the IMD. The messages sent by the transceiver module of IMD to the external device serve as response messages. Generally, messages are strings of bits embedded in a syntax and semantics system defined as part of a communication protocol expressed in and interpreted by well-defined algorithms and data structures. The request messages received by the IMDs transceiver module are relayed to the processor for data processing. Analogously, the processor produces the content of the signals/messages which are subsequently relayed to the transceiver module for sending to the external device as response messages.

The IMD may comprise further modules such as a memory for storing data, a power supply inclusive of battery support or otherwise, at least one sensor for retrieving physiological signals from the patient and/or a signal generator for generating and administering, for example, electrical or electromagnetic therapy signals. The transceiver module, the memory module, the power supply, the at least one sensor and/or the signal generator may be electrically connected to the processor.

The memory module of the IMD may include any volatile, non-volatile, magnetic, electrical media, or otherwise such as a random access memory (RAM), read-only memory (ROM), non-volatile RAM (NVRAM), electrically-erasable programmable ROM (EEPROM), flash memory, or any other memory storage type.

The external device is located at least partially extracorporeally and may be a separate module having a processor and may be wirelessly or by-wire connected to a computer (e.g. physician device, remote server, Programmer). Alternatively, the external device may form an integrated unit of such computer, for example may form an integrated unit of the Programmer. For bi-directional communication the external device may comprise a transceiver for messages (signals), for example, a coil connected to a communication module. The communication module may comprise or interfaces with a processor, in particular, if it is a module separate from a computer.

Wireless communication of the external device and the IMD comprises communication over the air (without wire). The communication may use inductive magnetic means, acoustic methods (e.g. ultrasound), and/or acoustic, optical and/or electromagnetic waves, for example Bluetooth, WLAN, ZigBee, NFC, Wibree or WiMAX in the radio frequency region, or IrDA or free-space optical communication (FSO) in the infrared or optical frequency region.

With regard to the invention, each processor is regarded as a functional unit of the IMD, the external device, and/or the computer, that interprets and executes instructions comprising an instruction control unit and an arithmetic and logic unit. The (remote) computer or external device is a functional unit that can perform substantial computations, including numerous arithmetic operations and logic operations without human intervention, such as, for example, a personal mobile device (PMD), a desktop computer, a server computer, clusters/warehouse scale computer or embedded system.

In one embodiment, the processor of the IMD adopts at least a predefined sleep state and a predefined active state. In the active state the processor collects, gathers, or ships internally stored data outbound (in one or more responses) or controls or takes actions to collect measurements or runs routines, e.g. for therapy application. Accordingly, in the active state, the processor operates for responding to external requests and/or for processing data according to an internal program sequence. In the following, the active state may represent a 'busy condition' which intentionally works to complete lengthy routines related to requests from the external device before cueing the system regarding the IMD's readiness for the processing of any further command requests. In the sleep state, however, the processor is only minimally engaged in support for communication (thereby saving electrical power) though the simple maintenance of the IMD in a condition where it is capable of being awakened for further active state interactions. It is only upon transitioning into a higher-power active state condition that the IMD is capable of facilitating further, targeted message/data exchange. In one embodiment the receiver circuitry is always running during the service time of the IMD as it must prove possible to communicate with the device whenever a health care provider (HCP) attempts interactions via the external device.

According to the invention, at the beginning of communication from the external device with the IMD, wherein the processor of the IMD is initially in the sleep state, the external device sends a predefined wake-up signal to the transceiver module of the IMD, wherein the wake-up signal is combined with an ID request message following the wake-up signal within a predefined first time interval, wherein the ID request message contains one of a predefined set of distinct request commands.

In one embodiment, the external device is configured to send a plurality of predefined wake-up signals to the transceiver module of the IMD, each combined with an ID request message following the wake-up signal within the predefined first time interval, wherein a second of such predefined wake-up signals follows the first of such predefined wake-up signals within a predefined second time interval, wherein the second time interval is longer than the first time interval. The first combined wake-up signal with ID request message may be treated analogously a known HEP (i.e. no noise). The first wake-up signal may be assessed by the IMD to assist in adjusting gain settings within the system for supporting further communication and the IMD may furthermore initiate a process to confirm that the observed signaling is in-fact a HEP input from an external device. On the next clustered grouping of wake-up signal and ID request message the IMD may confirm that the signals are in-fact valid wake-up inputs and then may proceed to initiate the communication clocking support and phase lock loop (PLL) elements necessary for decoding the baseline binary phase shift keying (BPSK) communication signaling. By the time the third grouping of wake-up signal and ID request message arrives at the implant, the transceiver of the IMD may be active and readied for further communication.

In one embodiment, the wake-up signal is an early 8-cycle pulse output train and a late 8-cycle pulse output train separated by an 8-cycle gap resulting in a 24-cycle long gapped output. Other wake-up signals may be used, too, including, for example, known two cycles of high energy clock pulse output (HEP), e.g. at 32.768 Hz as has been used in legacy product communication schemes. According to the first mentioned embodiment, the known, legacy product HEP implementation has been modified to enable a low-power wake-up for leadless support through the inclusion of greater clock cycle counts and an identifying internal gap signature. The increased cycle counts and gap feature improves deep-implant wake-up support without compromising device service time by enabling the IMD to consume less power in recognizing an incoming HEP as susceptibility to noise inputs can be more readily avoided. The above explained wake-up signal has the capability to be confirmed by a predefined pattern known by the processor or a transceiver circuitry of the IMD (i.e., matching the active pulses and gaps against a template) which may be used to ensure that wake-up of the processor (i.e. the transition from the sleep state to the active state) only occurs when intended - again, improving noise immunity. This revised deep-implant HEP wake-up approach furthermore maintains a capacity to awaken legacy IMDs, as well. In one embodiment, the pulse amplitudes of the early 8-cycle pulse output train may be different from the pulse amplitudes of the late 8-cycle pulse output train. This multi-level segmentation of the output within the HEP sequence offers added flexibilities for the IMD to determine what gain settings are best for subsequent baseline communication and to offer feedback to the external device regarding output amplitudes it should utilize during said subsequent baseline communication.

Alternatively or additionally, the wake-up signal is comprised of a pulse output train which may be segmented into an early segment and a late segment separated by an intervening gap, wherein, for example, the pulse amplitudes of the early segment of the pulse output train are the same or different when compared to the pulse amplitudes of the late segment of the pulse output train.

According to the invention, the ID request message following the wake-up signal represents one of a predefined set of distinct ID request commands which may be chosen randomly or in accordance with rule-based selection as part of the process involved in initiating baseline communication and the provisioning of time-multiplexed IMD responses. The ID request message contains information for the processor of the IMD to read the memory address within the memory module essential for appropriately handling the ID request message. For proper data exchange, the IMD needs to be within the external device's communication range. Based on the sent request specification, the processor of the IMD reads the information stored in command-relevant section of the memory module. This information is called "ID information". In one embodiment, the memory address is the memory address of the serial number of the IMD. In this embodiment, the ID information is a section of the IMD's serial number, wherein the ID request command determines, which section of the IMD's serial number has to or should be read by the IMD's processor.

In one embodiment, the external device chooses randomly or via rule-based measures one of the set of 8 ID request commands, wherein each request command directs the processor of the IMD to choose a different section of the memory, for example a three-bit-section of the memory. Rule-based choosing may include, for example, to choose each time the next ID request command in the family having a binary indexing value either one higher or one lower than the last used value or to choose every second higher or second lower value. If arrived at the highest or lowest value, the choosing continues at the lowest or highest value, respectively, i.e., wrapping. Other rules may be applied, as well.

Alternatively or additionally, external device is configured to randomly or in accordance with a rule-based scheme choose one of the set of a plurality of ID request commands, wherein each ID request command allows the processor of the IMD to choose a different section of the memory.

The ID information may be a certain value, for example a section of the serial number of the IMD. According to the invention, based on the read ID information the time slot is determined by the processor, in which the ID response message needs to be sent back to the external device. The probability is high, that for two different IMDs (each having, for example, a different serial number) two different ID information values will be derived from their respective memory modules. Accordingly, the ID response message of both IMDs will be sent by their transceivers using different time slots. This scheme is known as time-multiplexed response management. Thereby the external device is capable of distinguishing distinct IMDs. In a discovery phase application, such a time-multiplexing scheme allows a plurality of leadless IMDs to respond at prescribed delays relative to an ID request message to support communication with an external device. Additionally, it is ensured that recognition of any one IMD within the communication range of the external device does not hinder the ability for the system to interact with other IMDs or, worse yet, deny their visibility to the communication infrastructure altogether. The start of each time slot may represent a unique, fixed delay relative to the end of the ID request message and may have a fixed length. Each time slot may have the same or different length compared with the other time slots.

In one embodiment, the processor chooses one of 8 different time slots based on the ID information (for example a 3 bit code) read from the determined section of the IMD's memory. The ID response message of the IMD may contain the full or a part of the serial number of the IMD. Also more or less than 8 different time slots may be chosen by the processor.

Alternatively or additionally, the processor is configured to, based on the ID information read from the determined section of the memory, choose one of a plurality of time slots for sending the ID response message by the transceiver module.

Once IMDs are discovered as outlined above, the communication system may begin a follow-on process of pairing found IMDs with a short and unique reference address. The pairing process provided by the external device, may involve, for example, taking the read serial number (e.g., a 4-byte binary value) for any discovered IMD and assigning it a shorter (e.g., 3 bit binary value) and unique reference address. Such an approach means that any time messages are sent to one of a multitude of in-range IMDs, the process can proceed without having the message and response packets include the overhead of longer target addresses based on the serial number but instead simply including the shorter reference address. This approach leaves more room in the limited data throughput communication scheme used for leadless support to pass meaningful payload content throughout the communication link. It further enables improved clinical support and critical IMD change-out at end of service, effectively rendering a communication-based means for switching therapy output from one IMD to another. The inventive system and method allow focused leadless communication for data exchange, programming etc. with each IMD discovered in the range of the external device. Therein, the communication is not (necessarily) bound to specific time or location but may be provided anytime. Further, it is adapted to the specific energy requirements of small deep-implant leadless IMDs. The inventive method and IMD further eliminate the need to isolate individual IMDs from one another during production as part of factory program setting installation, debugging, calibration and other data relay needs.

According to the invention, an external device and an IMD both being a component of the communication system and having the above explained features solve the above problem, as well.

Analogously, the above problem is solved by a communication method for a wireless message transfer between an implantable medical device (IMD) and an external device, wherein the IMD monitors the health status of a patient and/or delivers a therapy signal to the patient, wherein the IMD comprises a processor, a memory module and a transceiver module which bi-directionally exchanges the messages with the external device, comprising the following steps:
- sending a predefined wake-up signal to the transceiver module of the IMD combined with an ID request message following the wake-up signal within a predefined first time interval by the external device, wherein the ID request message contains one of a predefined set of ID request commands,
- producing an ID response message to the previously received ID request message by the processor of the IMD in order to send this ID response message from the transceiver module to the external device within a time slot after the receipt of this ID request message, wherein the ID response message comprises an ID information read from a predefined memory address of the memory module, wherein the section of the memory is determined by the processor based on the previously received ID request message and the time slot is determined by the processor based on the ID information read from the determined section of the memory.

Alternatively or additionally, the ID response message comprises an ID information read from a memory cell section at a predefined memory address of the memory module, wherein the section of the memory is determined by the processor based on the ID request command sent by the previously received ID request message and the time slot is determined by the processor based on the ID information read from the determined section of the memory.

An ID request command may also be called request specification. A memory cell may also be called memory (without cell).

By the inventive method, a means is found for pulling minimal IMD status information (i.e. ID information) from any in-range (i.e., discovered) leadless IMD to aid in user selection of a specific targeted IMD for further interrogation/testing etc.

In one embodiment of the method, one of the set of 8 ID request command is randomly or via rule-based methodologies chosen by the external device, wherein each ID request command allows the processor of the IMD to choose a different section of the memory.

Alternatively or additionally, one of the multitude of ID request commands is randomly or in accordance with a rule-based scheme chosen by the external device, wherein each ID request command allows the processor of the IMD to choose a different section of the memory.

Further, in one embodiment, one of 8 different time slots for sending the ID response message is chosen by the processor based on the ID information read from the determined section of the memory.

Alternatively or additionally, one of a multitude of different time slots, e.g. 8 different time slots, for sending the ID response message is chosen by the processor based on the ID information read from the determined section of the memory.

Furthermore, in one embodiment, a plurality of predefined wake-up signals are sent by the external device to the transceiver module of the IMD, each combined with an ID request message following the wake-up signal within the predefined first time interval, wherein a second of such predefined wake-up signals follows the first of such predefined wake-up signals within a predefined second time interval, wherein the second time interval is longer than the first time interval.

The above method is, for example, realized as a computer program (to be executed at or within the external device and/or the IMD, in particular utilizing their processors) which is a combination of above and below specified (computer) instructions and data definitions that enable computer hardware or a communication system to perform computational or control functions and/or operations, or which is a syntactic unit that conforms to the rules of a particular programming language and that is composed of declarations and statements or instructions needed for an above and below specified function, task, or problem solution.

Furthermore, a computer program product is disclosed comprising instructions which, when executed by a processor, cause the processor to perform the steps of the above defined method. Accordingly, a computer readable data carrier storing such computer program product is described.

The present invention will now be described in further detail with reference to the accompanying schematic drawings, wherein
- Fig. 1: shows an embodiment of the inventive communication system comprising an implantable leadless pacemaker (ILP) and an external device, wherein the ILP is shown within a cross section of a patient's heart,
- Fig. 2: depicts a representation of one embodiment of a wake-up signal provided by the external device of Fig. 1,
- Fig. 3: shows an overview of the communication method of the external device and the ILP of Fig. 1 presented on a timeline,
- Fig. 4: a detailed arrangement of one embodiment of the wake-up signal, the ID request message and the time slots for the ID response message presented on a timeline, and
- Fig. 5: an example of a graphical user interface of a computer connected to the external device of Fig. 1 for pairing of discovered in-range ILPs.

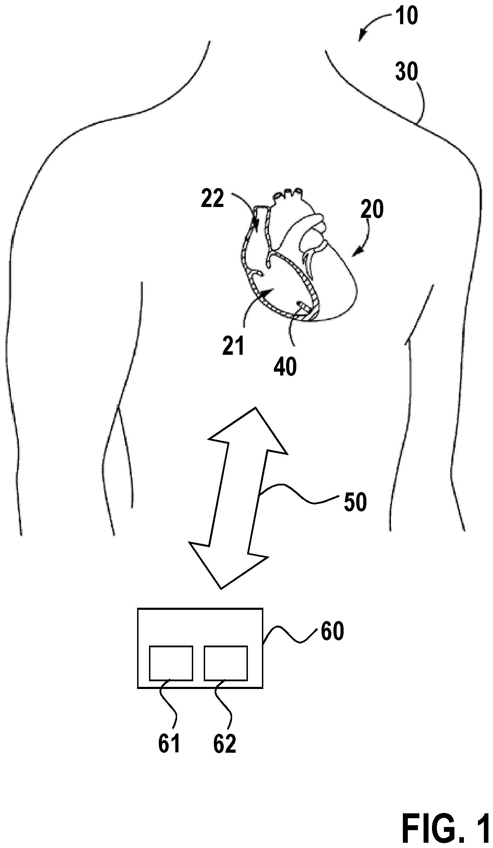

Fig. 1 shows an example communication system 10 and a heart 20 (with right ventricle 21 and right atrium 22) of a patient 30. The system 10 comprises a leadless ventricular pacemaker device 40 (hereinafter "ILP 40") as an example for an IMD and an external device 60. ILP 40 may be configured to be implanted within the right ventricle 21 of the heart 20 (as shown in Fig. 1) and to pace this ventricle and sense intrinsic ventricular depolarizations to inhibit ventricular pacing in response to said depolarizations. The ILP 40 may further comprise an accelerometer sensor in order to measure posture of the patient 30. A programmer (not shown) may be used to program ILP 40 using the external device 60. The external device 60 is located outside the body and is adapted to communicate bi-directionally with the ILP 40.

The ILP 40 may comprise modules such as a processor, a data memory module, a signal generator unit for providing treatment signals (e.g. pacing signals), a measurement unit comprising an ECG measuring unit, a DC impedance sensor and the accelerometer sensor, a transceiver for sending and receiving messages to the external device 60, and a power source - each of said modules being electrically connected in some fashion within the IMD. The power source may include a battery, (e.g., a rechargeable or non-rechargeable battery). The data memory module may include any memory type mentioned above. The processor of the ILP 40 may adopt at least the above explained sleep state and the above explained active state.

The external device 60 comprises a processor 61 and a transceiver 62 for exchange of messages with the ILP 40 which are electrically connected with each other. Further, the external device 60 may exchange data with other external devices and/or a remote server (not shown). The external device 60 may be the Programmer. The bi-directional exchange of messages with the ILP 40 is symbolized by the double-headed arrow 50. The leadless communication of external device 60 with the ILP 40 may be inductive magnetic communication, conducted communication, and acoustic communication, for example.

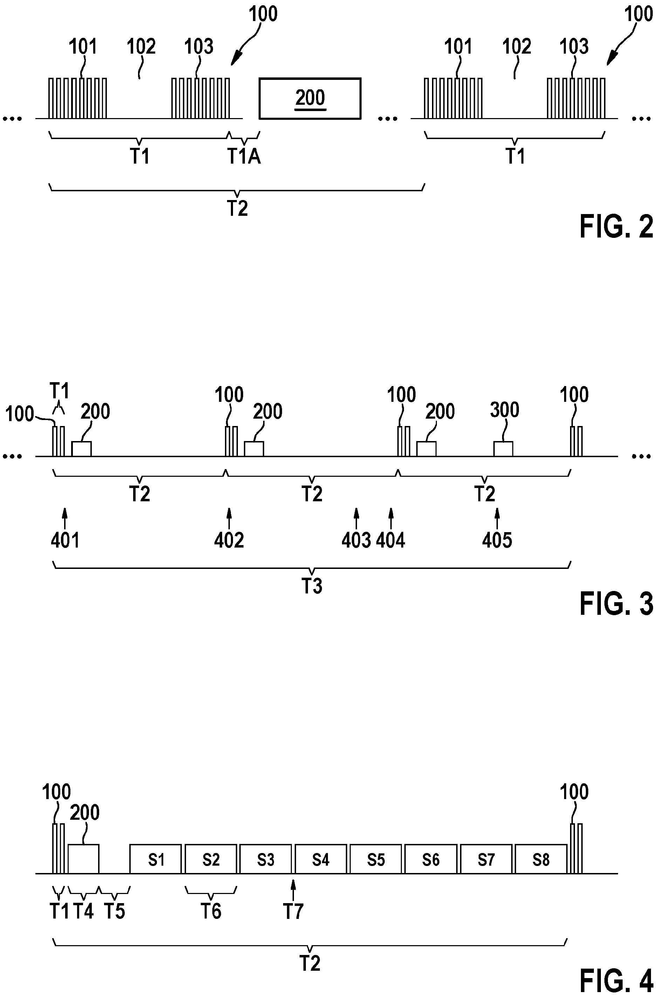

In the following, the operation of one embodiment of the communication system and method is explained with reference to Fig. 2 to 5.

To enable viable communication with multiple ILP 40 and to consider the greater implantation depths of such ILP 40, a wake-up signal 100 is provided which includes a more substantial count of clock cycles (compared to former sequence designs) as well as an identifying internal gap signature. Fig. 2 shows an example of such awake-up signal 100 consisting of two 8 cycle pulse output trains 101, 103 (i.e. one early pulse train 101 and a late pulse train 103) separated by an 8 cycle gap 102. Of course, a signal 100 with more or less cycles or cycle gaps, e.g. 6, 7, 9 or 10 cycles or cycle gaps, is possible. This 24 cycle-long gapped signal 100 shown Fig. 2 supports a lowering of the sensitivity necessary for the in-ILP receiver to recognize the wake-up signal 100. The wake-up signal 100 is repeated after a time interval T2 (for example T2 = 333 ms). The wake-up signal 100 has a length of T1 (for example T1 = 732 µs).

While the above-described wake-up signal discovers deep leadless implants, it still maintains a capacity to awaken state-of-the-art products for Programmer engagement. The above wake-up signal simply ends up being ignored or immaterial to such implant types as they require higher signal energy.

Fig. 3 and 4 contain a schematic representation for the wake-up signal 100 demonstrating how the wake-up signal used for leadless communication is paired with ID request messages to identify a plurality of leadless implants. For simplicity's sake Fig. 3 focuses on the start-up of communication with one single ILP 40 whose processor is initially in a sleep state explained above. After a short delay of a time interval T1A (see Fig. 2, for example T1A = 488 µs) following each wake-up signal 100 the external device 60 sends an ID request message 200 to the ILP 40 which is explained below in detail. Subject to initial wake-up signal 100 detection by the ILP 40, assessments may be made by the ILP of the gain conditions associated with receiver configuration for support of further communication and the processor of the ILP 40 initiates a process to confirm that observed signaling is in-fact a wake-up signal 100 (i.e., not noise) - a step represented by arrow 401. On the next clustered grouping of wake-up signals (after a time interval T2 of 333 ms in the shown embodiment) the transceiver of the ILP 40 confirms that the wake-up signal 100 with its pulse trains 101, 103 and their gap 102 is in-fact a valid wake-up signal (see step symbolized by arrow 402) and then proceeds to initiate the communication clocking support and phase lock loop (PLL) elements necessary for decoding the baseline binary phase shift keying (BPSK) communication signaling (step represented by arrow 403). By the time the third wake-up signal 100 arrives at the ILP, the processor of the ILP is in an active state (active state as explained above, step represented by arrow 404) and readied for further communication. The fourth wake-up signal may be initiated by the communication unit after a time interval T3 with regard to the beginning of the first wake-up signal (T3 = 3xT2 = 1s).

To support the initiation of baseline communication, after each wake-up signal 100 the external device 60 sends out an ID request message 200. This message when received by the ILP petitions the processor of the ILP 40 to reference the data memory module location where the ILP serial number has been stored using the request specification value contained in the ID request message 200. Based on the specific ID request message 200, in particular based on its request specification, a memory cell section of a small number of bits (e.g. 3 bits) is used to determine a specific time slot S1, S2, S3, S4, S5, S6, S7, S8 (see Fig. 4) in which the ILP will then send its ID response message 300 to the ID request command 200 (as shown in 405 of Fig. 3). Each time slot S1, S2, S3, S4, S5, S6, S7, S8 represent a specific delay relative to the ID request message 200 where the ILP 40 responds using its transceiver module to make its presence known and to carry its serial number (or a section of it) to the external device 60. In Fig. 3 a single ILP 40 is within range of the external device 60 and it responds in a devoted time slot (e.g. in time slot S5) simply labeled as a gray box tagged with reference number 300. After discovery of the single ILP 40 the individual communication with the external device 60 may start by, for example, transmitting the actual status of the ILP 40. The start of the baseline communication could begin following the point indicated by arrow 405 in Fig. 3. The table below further outlines the use of the request specification of the ID request messages 200. There are a plurality of request specifications or ID request commands for such message (8 in the specific shown embodiment, see first column of below table). Each determines bits from in-range ILP serial numbers stored in the data memory module of the respective ILP 40. The corresponding bits for each request specification are listed in the second column of below table. The value (which is called above ID information) stored in the prescribed bits of the serial number of any ILP within the range of the external device 60 encodes a specific time slot. As in the present embodiment three bits may have an ID information between 0 (binary: 000) and 8 (binary: 111) so that eight different time slots may be encoded as listed in the last column of below table. Each time slot S1 to S8 is depicted in Fig. 4 as a separate green box. The first time slot S1 begins a time interval T5 after the end of the ID request message 200, wherein T5 may be, for example 23.4 ms. The ID request command 200 may have a length T4 of 23.4 ms as well. The gap between each time slot may have a length of T7 = 488 µs.

| **Request Specification** | **Bits from Serial Number** | **Timeslot** |
|---|---|---|
| 0 x 10 | 0, 1, 2 | 000 = Timeslot 1 |
| 0 x 11 | 3, 4, 5 | 001 = Timeslot 2 |
| 0 x 12 | 6, 7, 8 | 010 = Timeslot 3 |
| 0 x 13 | 9, 10, 11 | 011 = Timeslot 4 |
| 0 x 14 | 12, 13, 14 | 100 = Timeslot 5 |
| 0 x 15 | 15, 16, 17 | 101 = Timeslot 6 |
| 0 x 16 | 18, 19, 20 | 110 = Timeslot 7 |
| 0 x 17 | 21, 22, 23 | 111 = Timeslot 8 |

Each time slot S1 to S8 represents a unique, fixed delay relative to the end of the ID request message 200. The delay has the length of T5 and several times the length T6 of each time slot and T7, wherein, for example, T6 = 35.5 ms. The delay for time slot S1 is T5, whereas the delay for time slot S2 is T5+T6+T7 OR T5 + the duration of S1 + a slight gap. For time slot S3 the delay amounts to T5 + 2xT6 + 2xT7 etc. OR T5 + the duration of S1 + a slight gap + the duration of S2 + a slight gap. The in-range ILP then each wait for a duration aligned with the time slot computed and then report their serial number as part of the ID response message 300. This scheme does not guarantee that for a given ID request message 200, all of the ILP 40 within range of the external device 60 will respond in a different time slot. In such cases, it is expected that overlapping responses from more than a single ILP in a shared time slot S1 to S8 garble the response recognition by the external device 60. In all likelihood, subject to this type of collision, one or both of the ILP cannot be discovered. However, each time an ID request message 200 is sent following a wake-up signal 100 by the external device 60, another request specification is chosen (of the 8 in the shown embodiment) and sent with the ID request message 200. The picking of the request specification may be carried out randomly or by a specific rule stored in the external device 60. The use of a new ID request command would "shuffle the deck", effectively picking another section of the in-range ILP serial numbers to assign time slots. This picking of ID request command to encode new time slots, statistically eventually allows for multiple ILP to land in separate, distinct time slots - facilitating their recognition by the external device 60.

The above-mentioned time-multiplexed scheme for external device recognition of individual ILP in prescribed time slots is a process which can be described as "Discovery". It is the only "frame-based" portion of the communication system (i.e. one where a prescribed regularity of message output is organized in time to provide allowances for responses in expected intervals). As can be derived from Fig. 4 by the short delay with the length T5 (for example 23.4 ms) between the ID request message 200 and the first time slot S1, the scheme also permits legacy IMDs to respond. In such cases since legacy IMDs were not designed to consider the possibility of having more than a single in-range IMD, they simply start communicating and, within this setup, they would be expected to override the system's attempts to engage with leadless IMDs, and the programmer software infrastructure would likely spawn an interface specific to such known legacy products. Accordingly, the inventive approach is capable of supporting communication with legacy IMDs. The ILP can be within the communication range of the external device, but it likely will not be "seen" by the external device if a legacy implant is also within its range. If one wishes to be certain one can communicate with an ILP, it is wise, if not necessary that legacy devices reside outside of the communication range of the external device.

Once ILPs are discovered as outlined above, the system may begin a follow-on process of pairing found ILPs with a short address. The pairing process involves taking the found serial number (e.g., a 4-byte binary value) for any discovered ILP and assigning it a shorter (e.g., 3 bit binary value) but unique reference address. Such an approach means that any time messages are sent to one of the plurality of in-range ILPs, the process can proceed without having the command and response packets include the overhead of longer target addresses based on the serial number. This approach leaves more room in the limited data throughput communication scheme used for leadless support to pass meaningful payload content throughout the communication system.

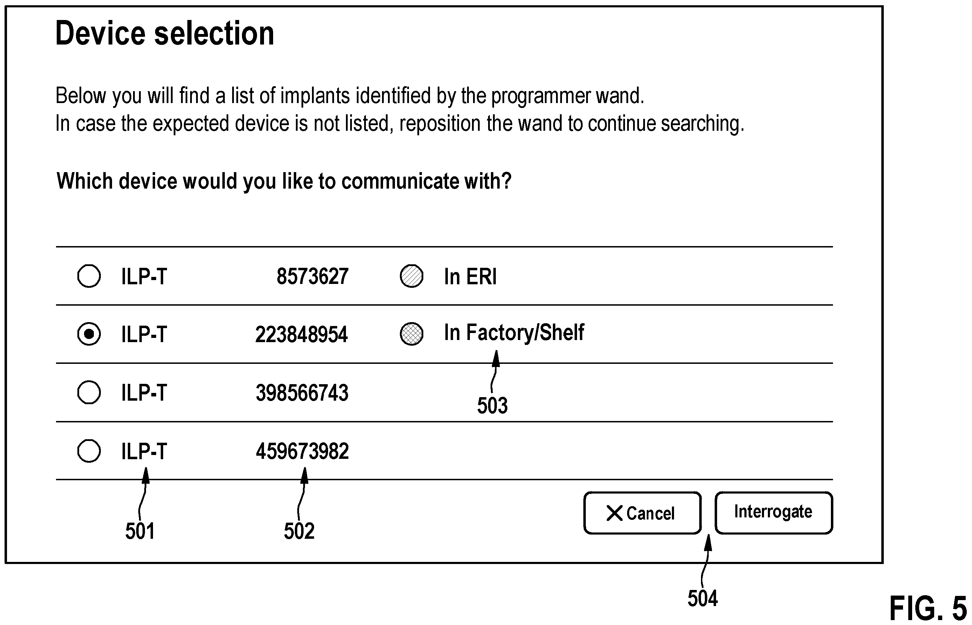

For any ILP that the system has paired with a short address, a status/type interrogation may then be run where the external device 60 requests that added information be reported. Basic information of this type may include, for example, whether or not the device resides in a factory/shelf state, what condition the device's battery presents, and/or a reporting of the ILP implant date - among other considerations. In a preferred embodiment, rather than using separate command-based interactions to poll for this status/type interrogation information, the content relayed by the IMD as part of the response to the ID request command may carry such information - this latter embodiment offering a lower overhead means to rapidly provide crucial device information to the system/users. The rendering of such details does not represent what traditionally has been known as a full device interrogation routine but instead a lighter touch pulling of targeted values from the in-range ILPs. Such interactions enable the generation of a user interface on graphical user interface (GUI) of the external device 60 that can assist health care providers in selecting the proper one of the plurality of ILPs found within the range of the external device 60. An example interface of this type is illustrated in Fig. 5 showing a list 501 of different ILP types within the external device's 60 range and their corresponding serial number (see list 502). Lists 501 and 503 contain examples of the types of light touch status information noted above. Following user selection of a specific ILP via GUI interaction, the communication system may enter a formal interrogation process and devoted baseline communications with the ILP of choice (in response to user instruction per, for example, interfacing through shown field 504).

## Claims

1. A communication system (10) for a wireless message transfer between an implantable medical device, IMD (40), and an external device (60), the communication system (10) comprising the IMD (40) and the external device (60), wherein the IMD (40) is configured to monitor the health status of a patient (30) and/or configured to deliver a therapy signal to the patient, wherein the IMD (40) comprises a processor, a memory module and a transceiver module configured to bi-directionally exchange the messages with the external device (60),
wherein the external device (60) is configured to send a predefined wake-up signal (100) to the transceiver module of the IMD (40) combined with an ID request message (200) following the wake-up signal within a predefined first time interval (T1A), wherein the ID request message contains one of a predefined set of predefined different request specifications,
wherein the processor of the IMD (40) is configured to produce an ID response message (300) to the previously received ID request message and to send this ID response message from the transceiver module to the external device (60) within a time slot after the receipt of this ID request message, wherein the ID response message comprises an ID information read from a predefined memory address of the memory module, wherein the section of the memory is determined by the processor based on the request specification sent by the previously received ID request message and the time slot (S1, S2, S3, S4, S5, S6, S7, S8) is determined by the processor based on the ID information read from the determined section of the memory.

2. The communication system of claim 1, wherein the memory address is the memory address of the serial number of the IMD (40).

3. The communication system of any of the previous claims, wherein external device (60) is configured to randomly or in accordance with a rule-based scheme choose one of the set of a plurality of ID request commands, wherein each ID request command allows the processor of the IMD (40) to choose a different section of the memory.

4. The communication system of any of the previous claims, wherein the processor is configured to, based on the ID information read from the determined section of the memory, choose one of a plurality of time slots (S1, S2, S3, S4, S5, S6, S7, S8, etc.) for sending the ID response message (300) by the transceiver module.

5. The communication system of any of the previous claims, wherein the external device (60) is configured to send a plurality of predefined wake-up signals (100) to the transceiver module of the IMD (40), each combined with an ID request message (200) following the wake-up signal within the predefined first time interval (T1A), wherein a second of such predefined wake-up signals follows the first of such predefined wake-up signals within a predefined second time interval (T2), wherein the second time interval is longer than the first time interval.

6. The communication system of any of the previous claims, wherein the wake-up signal (100) is comprised of a pulse output train which may be segmented into an early segment (101) and a late segment (103) separated by an intervening gap (102), wherein, for example, the pulse amplitudes of the early segment of the pulse output train are the same or different when compared to the pulse amplitudes of the late segment of the pulse output train.

7. The communication system of any of the previous claims, wherein the external device (60) is configured to assign each discovered IMD (40) within its range a short and unique reference address.

8. An external device (60) operable in a communication system (10) for a wireless message transfer between an IMD (40) and the external device (60), wherein the IMD (40) is configured to monitor the health status of a patient (30) and/or configured to deliver a therapy signal to the patient,
wherein the external device (60) is configured to send a predefined wake-up signal (100) to a transceiver module of the IMD (40) combined with an ID request message (200) following the wake-up signal within a predefined first time interval (T1A), wherein the ID request message contains one of a predefined set of predefined different request specifications, and
wherein the external device (60) is configured to receive an ID response message produced by the processor of the IMD (40) in response to the previously received ID request message and sent by the transceiver module of the IMD (40) within a time slot after the receipt of this ID request message, wherein the ID response message comprises an ID information read from a predefined memory address of the memory module of th IMD (40), wherein the section of the memory is determined by the processor of the IMD (40) based on the request specification sent by the previously received ID request message from the external device (60) and the time slot (S1, S2, S3, S4, S5, S6, S7, S8) is determined by the processor of the IMD (40) based on the ID information read from the determined section of the memory of the IMD (40).

9. An implantable medical device, IMD (40), operable in a communication system (10) for a wireless message transfer between the IMD (40) and an external device (60), wherein the IMD (40) is configured to monitor the health status of a patient (30) and/or configured to deliver a therapy signal to the patient,
wherein the IMD (40) comprises a processor, a memory module and a transceiver module configured to bi-directionally exchange the messages with the external device (60),
wherein the processor of the IMD (40) is configured to produce an ID response message (300) to an ID request message reveived via the transceiver module from the external device (60) and to send this ID response message from the transceiver module to the external device (60) within a time slot after the receipt of this ID request message,
wherein the ID request message contains one of a predefined set of predefined different request specifications,
wherein the ID response message comprises an ID information read from a predefined memory address of the memory module,
wherein the section of the memory is determined by the processor based on the request specification sent by the previously received ID request message and the time slot (S1, S2, S3, S4, S5, S6, S7, S8) is determined by the processor based on the ID information read from the determined section of the memory.

10. A communication method for a wireless message transfer between an implantable medical device, IMD (40), and an external device (60), wherein the IMD (40) monitors the health status of a patient (30) and/or delivers a therapy signal to the patient, wherein the IMD (40) comprises a processor, a memory module and a transceiver module which bi-directionally exchanges the messages with the external device (60), comprising the following steps:
- sending a predefined wake-up signal (100) to the transceiver module of the IMD (40) combined with an ID request message (200) following the wake-up signal within a predefined first time interval (T1A) by the external device (60), wherein the ID request message contains one of a predefined set of predefined different ID request commands,
- producing an ID response message (300) to the previously received ID request message by the processor of the IMD (40) in order to send this ID response message from the transceiver module to the external device (60) within a time slot after the receipt of this ID request message, wherein the ID response message comprises an ID information read from a memory cell section at a predefined memory address of the memory module, wherein the section of the memory is determined by the processor based on the ID request command sent by the previously received ID request message and the time slot (S1, S2, S3, S4, S5, S6, S7, S8) is determined by the processor based on the ID information read from the determined section of the memory.

11. The communication method of claim 10, wherein one of the multitude of ID request commands is randomly or in accordance with a rule-based scheme chosen by the external device (60), wherein each ID request command allows the processor of the IMD (40) to choose a different section of the memory.

12. The communication method of any of the claims 10 to 11, wherein one of a multitude of different time slots (S1, S2, S3, S4, S5, S6, S7, S8, etc.) for sending the ID response message (300) is chosen by the processor based on the ID information read from the determined section of the memory.

13. The communication method of any of the claims 10 to 12, wherein a plurality of predefined wake-up signals (100) are sent by the external device (60) to the transceiver module of the IMD (40), each combined with an ID request message (200) following the wake-up signal within the predefined first time interval (T1A), wherein a second of such predefined wake-up signals follows the first of such predefined wake-up signals within a predefined second time interval (T2), wherein the second time interval is longer than the first time interval.

14. A computer program product comprising instructions which, when executed by a processor, cause the processor to perform the steps of the method according to any of the claims 10 to 13.

15. Computer readable data carrier storing a computer program product according to claim 14.

## Patentansprüche

1. Kommunikationssystem (10) für eine drahtlose Nachrichtenübermittlung zwischen einer implantierbaren medizinischen Vorrichtung, IMD (40), und einer externen Vorrichtung (60), wobei das Kommunikationssystem (10) die IMD (40) und die externe Vorrichtung (60) umfasst, wobei die IMD (40) dafür konfiguriert ist, den Gesundheitszustand eines Patienten (30) zu überwachen, und/oder dafür konfiguriert ist, ein therapeutisches Signal an den Patienten zu liefern, wobei die IMD (40) einen Prozessor, ein Speichermodul und ein Transceiver-Modul umfasst, dafür konfiguriert, bidirektional die Nachrichten mit der externen Vorrichtung (60) auszutauschen,
wobei die externe Vorrichtung (60) dafür konfiguriert ist, ein vordefiniertes Aktivierungssignal (100) an das Transceiver-Modul der IMD (40) zu senden, in Kombination mit einer ID-Anfragenachricht (200), die innerhalb eines vordefinierten ersten Zeitintervalls (T1A) auf das Aktivierungssignal folgt, wobei die ID-Anfragenachricht eine von einem vordefinierten Satz vordefinierter verschiedener Anfragespezifikationen enthält,
wobei der Prozessor der IMD (40) dafür konfiguriert ist, eine ID-Antwortnachricht (300) auf die zuvor erhaltene ID-Anfragenachricht zu erzeugen und diese ID-Antwortnachricht von dem Transceiver-Modul an die externe Vorrichtung (60) innerhalb eines Zeitfensters nach Erhalt dieser ID-Anfragenachricht zu senden, wobei die ID-Antwortnachricht eine aus einer vordefinierten Speicheradresse des Speichermoduls ausgelesene ID-Information umfasst, wobei der Abschnitt des Speichers von dem Prozessor auf Grundlage der Anfragespezifikation bestimmt wird, die von der zuvor erhaltenen ID-Anfragenachricht gesendet wurde, und das Zeitfenster (S1, S2, S3, S4, S5, S6, S7, S8) von dem Prozessor auf Grundlage der aus dem bestimmten Abschnitt des Speichers ausgelesenen ID-Information bestimmt wird.

2. Kommunikationssystem nach Anspruch 1, wobei die Speicheradresse die Speicheradresse der Seriennummer der IMD (40) ist.

3. Kommunikationssystem nach einem der vorstehenden Ansprüche, wobei die externe Vorrichtung (60) dafür konfiguriert ist, zufällig oder gemäß einem regelbasierten Ansatz einen aus dem Satz einer Vielzahl von ID-Anfragebefehlen auszuwählen, wobei jeder ID-Anfragebefehl dem Prozessor der IMD (40) erlaubt, einen anderen Abschnitt des Speichers auszuwählen.

4. Kommunikationssystem nach einem der vorstehenden Ansprüche, wobei der Prozessor dafür konfiguriert ist, auf Grundlage der aus dem bestimmten Abschnitt des Speichers ausgelesenen ID-Information eins aus einer Vielzahl von Zeitfenstern (S1, S2, S3, S4, S5, S6, S7, S8 usw.) auszuwählen, um die ID-Antwortnachricht (300) durch das Transceiver-Modul zu senden.

5. Kommunikationssystem nach einem der vorstehenden Ansprüche, wobei die externe Vorrichtung (60) dafür konfiguriert ist, eine Vielzahl vordefinierter Aktivierungssignale (100) an das Transceiver-Modul der IMD (40) zu senden, jeweils in Kombination mit einer ID-Anfragenachricht (200) die innerhalb eines vordefinierten ersten Zeitintervalls (T1A) auf das Aktivierungssignal folgt, wobei ein zweites solcher vordefinierten Aktivierungssignale innerhalb eines vordefinierten zweiten Zeitintervalls (T2) auf das erste solcher vordefinierten Aktivierungssignale folgt, wobei das zweite Zeitintervall länger als das erste Zeitintervall ist.

6. Kommunikationssystem nach einem der vorstehenden Ansprüche, wobei das Aktivierungssignal (100) aus einer Impuls-Ausgabefolge besteht, die in ein frühes Segment (101) und ein spätes Segment (103), getrennt durch eine Lücke (102), segmentiert sein kann, wobei zum Beispiel die Impulsamplituden des frühen Segments der Impulsausgabefolge im Vergleich zu den Impulsamplituden des späten Segments der Impulsausgabefolge gleich oder unterschiedlich sind.

7. Kommunikationssystem nach einem der vorstehenden Ansprüche, wobei die externe Vorrichtung (60) dafür konfiguriert ist, jeder erkannten IMD (40) innerhalb ihres Bereichs eine kurze und einzigartige Referenzadresse zuzuweisen.

8. Externe Vorrichtung (60), die in einem Kommunikationssystem (10) für eine drahtlose Nachrichtenübermittlung zwischen einer IMD (40) und dem externen Gerät (60) betriebsfähig ist, wobei die IMD (40) dafür konfiguriert ist, den Gesundheitszustand eines Patienten (30) zu überwachen, und/oder dafür konfiguriert ist, ein therapeutisches Signal an den Patienten zu liefern,
wobei die externe Vorrichtung (60) dafür konfiguriert ist, ein vordefiniertes Aktivierungssignal (100) an ein Transceiver-Modul der IMD (40) zu senden, in Kombination mit einer ID-Anfragenachricht (200), die innerhalb eines vordefinierten ersten Zeitintervalls (T1A) auf das Aktivierungssignal folgt, wobei die ID-Anfragenachricht eine von einem vordefinierten Satz vordefinierter verschiedener Anfragespezifikationen enthält, und
wobei die externe Vorrichtung (60) dafür konfiguriert ist, eine ID-Antwortnachricht zu erhalten, die von dem Prozessor der IMD (40) als Antwort auf die zuvor erhaltene ID-Anfragenachricht erzeugt und vom Transceiver-Modul der IMD (40) innerhalb eines Zeitfensters nach dem Erhalt dieser ID-Anfragenachricht gesendet wird, wobei die ID-Antwortnachricht eine aus einer vordefinierten Speicheradresse des Speichermoduls der IMD (40) ausgelesene ID-Information umfasst, wobei der Abschnitt des Speichers von dem Prozessor der IMD (40) auf Grundlage der Anfragespezifikation bestimmt wird, die von der zuvor erhaltenen ID-Anfragenachricht von der externen Vorrichtung (60) gesendet wurde, und das Zeitfenster (S1, S2, S3, S4, S5, S6, S7, S8) von dem Prozessor der IMD (40) auf Grundlage der aus dem bestimmten Abschnitt des Speichers der IMD (40) ausgelesenen ID-Information bestimmt wird.

9. Implantierbare medizinische Vorrichtung, IMD (40), die in einem Kommunikationssystem (10) für eine drahtlose Nachrichtenübermittlung zwischen der IMD (40) und einer externen Vorrichtung (60) betriebsfähig ist, wobei die IMD (40) dafür konfiguriert ist, den Gesundheitszustand eines Patienten (30) zu überwachen, und/oder dafür konfiguriert ist, ein therapeutisches Signal an den Patienten zu liefern, wobei die IMD (40) einen Prozessor, ein Speichermodul und ein Transceiver-Modul umfasst, dafür konfiguriert, bidirektional die Nachrichten mit der externen Vorrichtung (60) auszutauschen,
wobei der Prozessor der IMD (40) dafür konfiguriert ist, eine ID-Antwortnachricht (300) auf die zuvor über das Transceiver-Modul von der externen Vorrichtung (60) erhaltene ID-Anfragenachricht zu erzeugen und diese ID-Antwortnachricht von dem Transceiver-Modul an die externe Vorrichtung (60) innerhalb eines Zeitfensters nach dem Erhalt dieser ID-Anfragenachricht zu senden,
wobei die ID-Anfragenachricht eine von einem vordefinierten Satz vordefinierter verschiedener Anfragespezifikationen enthält,
wobei die ID-Antwortnachricht eine aus einer vordefinierten Speicheradresse des Speichermoduls ausgelesene ID-Information umfasst,
wobei der Abschnitt des Speichers von dem Prozessor auf Grundlage der Anfragespezifikation bestimmt wird, die von der zuvor erhaltenen ID-Anfragenachricht gesendet wurde, und das Zeitfenster (S1, S2, S3, S4, S5, S6, S7, S8) von dem Prozessor auf Grundlage der aus dem bestimmten Abschnitt des Speichers ausgelesenen ID-Information bestimmt wird.

10. Kommunikationsverfahren für eine drahtlose Nachrichtenübermittlung zwischen einer implantierbaren medizinischen Vorrichtung, IMD (40), und einer externen Vorrichtung (60), wobei die IMD (40) den Gesundheitszustand eines Patienten (30) überwacht, und/oder ein therapeutisches Signal an den Patienten liefert, wobei die IMD (40) einen Prozessor, ein Speichermodul und ein Transceiver-Modul umfasst, das bidirektional die Nachrichten mit der externen Vorrichtung (60) austauscht, die folgenden Schritte umfassend:
- Senden eines vordefinierten Aktivierungssignals (100) an das Transceiver-Modul der IMD (40), in Kombination mit einer ID-Anfragenachricht (200), die innerhalb eines vordefinierten ersten Zeitintervalls (T1A) auf das Aktivierungssignal folgt, durch die externe Vorrichtung (60), wobei die ID-Anfragenachricht einen von einem vordefinierten Satz vordefinierter verschiedener ID-Anfragebefehle enthält,
- Erzeugen einer ID-Antwortnachricht (300) auf die zuvor erhaltene ID-Anfragenachricht durch den Prozessor der IMD (40), um diese ID-Antwortnachricht von dem Transceiver-Modul an die externe Vorrichtung (60) innerhalb eines Zeitfensters nach Erhalt dieser ID-Anfragenachricht zu senden, wobei die ID-Antwortnachricht eine aus einem Abschnitt der Speicherzelle an einer vordefinierten Speicheradresse des Speichermoduls ausgelesene ID-Information umfasst, wobei der Abschnitt des Speichers von dem Prozessor auf Grundlage des ID-Anfragebefehls bestimmt wird, der von der zuvor erhaltenen ID-Anfragenachricht gesendet wurde, und das Zeitfenster (S1, S2, S3, S4, S5, S6, S7, S8) von dem Prozessor auf Grundlage der aus dem bestimmten Abschnitt des Speichers ausgelesenen ID-Information bestimmt wird.

11. Kommunikationsverfahren nach Anspruch 10, wobei einer von der Vielzahl von ID-Anfragebefehlen zufällig oder gemäß einem regelbasierten Ansatz durch die externe Vorrichtung (60) ausgewählt wird, wobei jeder ID-Anfragebefehl dem Prozessor der IMD (40) erlaubt, einen anderen Abschnitt des Speichers auszuwählen.

12. Kommunikationsverfahren nach einem der Ansprüche 10 bis 11, wobei eins von einer Vielzahl von Zeitfenstern (S1, S2, S3, S4, S5, S6, S7, S8 usw.), um die ID-Antwortnachricht (300) zu senden, auf Grundlage der aus dem bestimmten Abschnitt des Speichers ausgelesenen ID-Information von dem Prozessor ausgewählt wird.

13. Kommunikationsverfahren nach einem der Ansprüche 10 bis 12, wobei eine Vielzahl vordefinierter Aktivierungssignale (100) von der externen Vorrichtung (60) an das Transceiver-Modul der IMD (40) gesendet wird, jeweils in Kombination mit einer ID-Anfragenachricht (200), die innerhalb eines vordefinierten ersten Zeitintervalls (T1A) auf das Aktivierungssignal folgt, wobei ein zweites solcher vordefinierten Aktivierungssignale innerhalb eines vordefinierten zweiten Zeitintervalls (T2) auf das erste solcher vordefinierten Aktivierungssignale folgt, wobei das zweite Zeitintervall länger als das erste Zeitintervall ist.

14. Computerprogrammprodukt, Anweisungen umfassend, die bei Ausführung durch einen Prozessor den Prozessor veranlassen, die Schritte des Verfahrens nach einem der Ansprüche 10 bis 13 auszuführen.

15. Computerlesbarer Datenträger, auf dem ein Computerprogrammprodukt nach Anspruch 14 gespeichert ist.

## Revendications

1. Système de communication (10) destiné à un transfert de messages sans fil entre un dispositif médical implantable ou IMD (40) et un dispositif externe (60), le système de communication (10) comprenant l'IMD (40) et le dispositif externe (60), dans lequel l'IMD (40) est configuré pour surveiller l'état de santé d'un patient (30) et/ou configuré pour délivrer un signal de traitement au patient, dans lequel l'IMD (40) comprend un processeur, un module de mémoire et un module émetteur-récepteur configuré pour échanger de manière bidirectionnelle les messages avec le dispositif externe (60),
dans lequel le dispositif externe (60) est configuré pour envoyer un signal de sortie de veille prédéfini (100) au module émetteur-récepteur de l'IMD (40) combiné avec un message de requête d'ID (200) suivant le signal de sortie de veille dans un premier intervalle de temps prédéfini (T1A), dans lequel le message de requête d'ID contient une parmi un ensemble prédéfini de spécifications de requête différentes prédéfinies,
dans lequel le processeur de l'IMD (40) est configuré pour produire un message de réponse d'ID (300) au message de requête d'ID reçu précédemment et pour envoyer ledit message de réponse d'ID du module émetteur-récepteur au dispositif externe (60) dans une plage de temps après la réception dudit message de requête d'ID, dans lequel le message de réponse d'ID comprend des informations d'ID lues depuis une adresse mémoire prédéfinie du module de mémoire, dans lequel la section de la mémoire est déterminée par le processeur en se basant sur la spécification de requête envoyée par le message de requête d'ID précédemment reçu et la plage temporelle (S1, S2, S3, S4, S5, S6, S7, S8) est déterminée par le processeur en se basant sur les informations d'ID lues depuis la section déterminée de la mémoire.

2. Système de communication selon la revendication 1, dans lequel l'adresse mémoire est l'adresse mémoire du numéro de série de l'IMD (40).

3. Système de communication selon l'une quelconque des revendications précédentes, dans lequel le dispositif externe (60) est configuré pour, aléatoirement ou conformément à un schéma basé sur des règles, choisir une parmi l'ensemble d'une pluralité de commandes de requête d'ID, dans lequel chaque commande de requête d'ID permet au processeur de l'IMD (40) de choisir une section différente de la mémoire.

4. Système de communication selon l'une quelconque des revendications précédentes, dans lequel le processeur est configuré pour, en se basant sur les informations d'ID lues depuis la section déterminée de la mémoire, choisir une parmi une pluralité de plages temporelles (S1, S2, S3, S4, S5, S6, S7, S8, etc.) pour faire envoyer le message de réponse d'ID (300) par le module émetteur-récepteur.

5. Système de communication selon l'une quelconque des revendications précédentes, dans lequel le dispositif externe (60) est configuré pour envoyer une pluralité de signaux de sortie de veille prédéfinis (100) au module émetteur-récepteur de l'IMD (40), chacun étant combiné à un message de requête d'ID (200) suivant le signal de sortie de veille dans le premier intervalle de temps prédéfini (T1A), dans lequel un deuxième de tels signaux de sortie de veille prédéfinis suit le premier de tels signaux de sortie de veille prédéfinis dans un deuxième intervalle de temps prédéfini (T2), dans lequel le deuxième intervalle de temps est plus long que le premier intervalle de temps.

6. Système de communication selon l'une quelconque des revendications précédentes, dans lequel le signal de sortie de veille (100) est constitué d'un train de sorties d'impulsion qui peut être segmenté en un segment précoce (101) et un segment tardif (103) séparés par un intervalle intermédiaire (102), dans lequel, par exemple, les amplitudes d'impulsion du segment précoce du train de sorties d'impulsion sont identiques ou différentes par comparaison aux amplitudes d'impulsion du segment tardif du train de sorties d'impulsion.

7. Système de communication selon l'une quelconque des revendications précédentes, dans lequel le dispositif externe (60) est configuré pour attribuer à chaque IMD (40) découverte à sa portée une adresse de référence courte et unique.

8. Dispositif externe (60) pouvant fonctionner dans un système de communication (10) destiné à un transfert de messages sans fil entre un IMD (40) et le dispositif externe (60), dans lequel l'IMD (40) est configuré pour surveiller l'état de santé d'un patient (30) et/ou configuré pour délivrer un signal de traitement au patient,
dans lequel le dispositif externe (60) est configuré pour envoyer un signal de sortie de veille prédéfini (100) à un module émetteur-récepteur de l'IMD (40) combiné avec un message de requête d'ID (200) suivant le signal de sortie de veille dans un premier intervalle de temps prédéfini (T1A),
dans lequel le message de requête d'ID contient une parmi un ensemble prédéfini de spécifications de requête différentes prédéfinies, et
dans lequel le dispositif externe (60) est configuré pour recevoir un message de réponse d'ID produit par le processeur de l'IMD (40) en réponse au message de requête d'ID reçu précédemment et envoyé par le module émetteur-récepteur de l'IMD (40) dans une plage temporelle après la réception dudit message de requête d'ID, dans lequel le message de réponse d'ID comprend des informations d'ID lues depuis une adresse mémoire prédéfinie du module de mémoire de l'IMD (40), dans lequel la section de la mémoire est déterminée par le processeur de l'IMD (40) en se basant sur la spécification de requête envoyée par le message de requête d'ID précédemment reçu depuis le dispositif externe (60) et la plage temporelle (S1, S2, S3, S4, S5, S6, S7, S8) est déterminée par le processeur de l'IMD (40) en se basant sur les informations d'ID lues depuis la section déterminée de la mémoire de l'IMD (40).

9. Dispositif médical implantable, ou IMD (40), pouvant fonctionner dans un système de communication (10) destiné à un transfert de messages sans fil entre l'IMD (40) et un dispositif externe (60), dans lequel l'IMD (40) est configuré pour surveiller l'état de santé d'un patient (30) et/ou configuré pour délivrer un signal de traitement au patient, dans lequel l'IMD (40) comprend un processeur, un module de mémoire et un module émetteur-récepteur configuré pour échanger de manière bidirectionnelle les messages avec le dispositif externe (60),
dans lequel le processeur de l'IMD (40) est configuré pour produire un message de réponse d'ID (300) à un message de requête d'ID reçu par l'intermédiaire du module émetteur-récepteur depuis le dispositif externe (60) et pour envoyer ledit message de réponse d'ID du module émetteur-récepteur au dispositif externe (60) dans une plage temporelle après la réception dudit message de requête d'ID,
dans lequel le message de requête d'ID contient une parmi un ensemble prédéfini de spécifications de requête différentes prédéfinies,
dans lequel le message de réponse d'ID comprend des informations d'ID lues depuis une adresse mémoire prédéfinie du module de mémoire,
dans lequel la section de la mémoire est déterminée par le processeur en se basant sur la spécification de requête envoyée par le message de requête d'ID précédemment reçu et la plage temporelle (S1, S2, S3, S4, S5, S6, S7, S8) est déterminée par le processeur en se basant sur les informations d'ID lues depuis la section déterminée de la mémoire.

10. Méthode de communication destinée à un transfert de messages sans fil entre un dispositif médical implantable ou IMD (40) et un dispositif externe (60), dans laquelle l'IMD (40) surveille l'état de santé d'un patient (30) et/ou délivre un signal de traitement au patient, dans laquelle l'IMD (40) comprend un processeur, un module de mémoire et un module émetteur-récepteur qui échange de manière bidirectionnelle les messages avec le dispositif externe (60), comprenant les étapes suivantes consistant à :
- envoyer un signal de sortie de veille prédéfini (100) au module émetteur-récepteur de l'IMD (40) combiné avec un message de requête d'ID (200) suivant le signal de sortie de veille dans un premier intervalle de temps prédéfini (T1A) par le dispositif externe (60), dans lequel le message de requête d'ID contient une parmi un ensemble prédéfini de commandes de requête d'ID différentes prédéfinies,
- produire un message de réponse d'ID (300) au message de requête d'ID reçu précédemment par le processeur de l'IMD (40) afin d'envoyer ledit message de réponse d'ID du module émetteur-récepteur au dispositif externe (60) dans une plage de temps après la réception dudit message de requête d'ID, dans lequel le message de réponse d'ID comprend des informations d'ID lues depuis une section de cellule de mémoire à une adresse mémoire prédéfinie du module de mémoire, dans lequel la section de la mémoire est déterminée par le processeur en se basant sur la commande de requête d'ID envoyée par le message de requête d'ID précédemment reçu et la plage temporelle (S1, S2, S3, S4, S5, S6, S7, S8) est déterminée par le processeur en se basant sur les informations d'ID lues depuis la section déterminée de la mémoire.

11. Méthode de communication selon la revendication 10, dans laquelle une parmi la multitude de commandes de requête d'ID est, aléatoirement ou conformément à un schéma basé sur des règles, choisie par le dispositif externe (60), dans laquelle chaque commande de requête d'ID permet au processeur de l'IMD (40) de choisir une section différente de la mémoire.

12. Méthode de communication selon l'une quelconque des revendications 10 à 11, dans laquelle une parmi une multitude de plages temporelles (S1, S2, S3, S4, S5, S6, S7, S8, etc.) différentes destinées à envoyer le message de réponse d'ID (300) est choisie par le processeur en se basant sur les informations d'ID lues depuis la section déterminée de la mémoire.

13. Méthode de communication selon l'une quelconque des revendications 10 à 12, dans laquelle une pluralité de signaux de sortie de veille prédéfinis (100) sont envoyés par le dispositif externe (60) au module émetteur-récepteur de l'IMD (40), chacun étant combiné à un message de requête d'ID (200) suivant le signal de sortie de veille dans le premier intervalle de temps prédéfini (T1A), dans lequel un deuxième de tels signaux de sortie de veille prédéfinis suit le premier de tels signaux de sortie de veille prédéfinis dans un deuxième intervalle de temps prédéfini (T2), dans lequel le deuxième intervalle de temps est plus long que le premier intervalle de temps.

14. Produit de programme informatique comprenant des instructions qui, lorsqu'elles sont exécutées par un processeur, amènent le processeur à réaliser les étapes de la méthode selon l'une quelconque des revendications 10 à 13.

15. Support de données lisible par ordinateur stockant un produit de programme informatique selon la revendication 14.
